# EUROPÄISCHE PATENTSCHRIFT

(11) **EP 3 659 659 B1**
(45) Veröffentlichungstag und Bekanntmachung des Hinweises auf die Patenterteilung: **28.07.2021**
(21) Anmeldenummer: 19211181.3
(22) Anmeldetag: 25.11.2019
(51) Int. Cl.: H05B 3/04, H05B 3/26, A24F 40/46, A24F 40/70, A61M 11/04, A61M 15/06

(54) **VERFAHREN ZUR HERSTELLUNG EINER MEHRZAHL VON ELEKTRISCH BEHEIZBAREN HEIZKÖRPERN FÜR EINE VERDAMPFERVORRICHTUNG FÜR EINEN INHALATOR**
METHOD FOR PRODUCING A PLURALITY OF ELECTRICALLY HEATABLE HEATING ELEMENTS FOR A VAPORIZING DEVICE FOR AN INHALER
PROCÉDÉ DE FABRICATION D'UNE PLURALITÉ D'ÉLÉMENTS CHAUFFANTS CHAUFFABLE ÉLECTRIQUEMENT POUR UN DISPOSITIF D'ÉVAPORATION POUR UN INHALATEUR

(30) Priorität: 28.11.2018 DE 102018130106
(43) Veröffentlichungstag der Anmeldung: 03.06.2020
(73) Patentinhaber: Hauni Maschinenbau GmbH, 21033 Hamburg (DE)
(72) Erfinder: KESSLER, Marc, 22415 Hamburg (DE); SCHMIDT, Markus, 21502 Geesthacht (DE)
(74) Vertreter: Müller Verweyen

(56) Entgegenhaltungen:
- WO-A1-2017/220274
- CN-U- 204 499 489
- DE-A1-102016 120 803
- DE-A1-102017 111 119
- US-A1- 2006 196 968
- US-A1- 2016 345 630
- US-A1- 2017 106 113

## Beschreibung

Die vorliegende Erfindung betrifft ein Verfahren zur Herstellung einer Mehrzahl von elektrisch beheizbaren Heizkörpern für eine Verdampfervorrichtung für einen Inhalator.

Herkömmliche Inhalatoren basieren auf der Docht-Wendel-Technologie. Durch Kapillarkräfte wird die Flüssigkeit aus einem Flüssigkeitsspeicher entlang eines Dochts so weit transportiert, bis die Flüssigkeit durch einen elektrisch beheizbaren Wendel erhitzt und somit verdampft wird. Die Docht-Wendel-Technologie hat jedoch den Nachteil, dass eine mangelnde Versorgung mit Flüssigkeit zu einer lokalen Überhitzung führen kann, wodurch Schadstoffe freigegeben werden können. Dies ist der sogenannte "Dry Puff" und diesen gilt es zu vermeiden.

Bei einer im Stand der Technik bekannten Verdampfervorrichtungen wird Flüssigkeit aus einem Flüssigkeitsspeicher zu der Durchgangsöffnung transportiert, wo die Flüssigkeit erwärmt und verdampft wird. Der im Gegensatz zur Docht-Wendel-Technologie andere Transport von Flüssigkeit kann zu Leckage führen.

Der Heizkörper ist von dem Träger gehalten und dadurch sind der Heizkörper und der Träger miteinander mechanisch verbunden. Daher wird die Wärme des Heizkörpers in den Träger abgeleitet. Die in den Träger abgeleitete Wärme kann die mechanischen Eigenschaften des Trägers nachteilig beeinflussen und/oder das Nutzererlebnis beeinträchtigen. Zudem stellt die Wärmeableitung beziehungsweise der Wärmetransport vom Heizkörper in den Träger eine unnötige energetische Belastung für einen elektrischen Energiespeicher des Inhalators dar.

US 2016/0345630 A1 offenbart eine Verdampfervorrichtung für ein elektronisches Zigarettenprodukt mit einem Heizkörper, einem Träger zur Halterung des Heizkörpers und einer zwischen dem Heizkörper und dem Träger angeordneten Dichtungsvorrichtung.

DE 10 2017 111 119 A1 und DE 10 2016 120 803 A1 offenbaren weitere Verdampfervorrichtungen für ein elektronisches Zigarettenprodukt.

Es ist die Aufgabe der Erfindung, ein Herstellungsverfahren für eine Verdampfervorrichtung bereitzustellen.

Die Aufgabe wird gelöst durch die Merkmale des unabhängigen Anspruchs 1.

Das erfindungsgemäße Verfahren zur Herstellung einer Mehrzahl von elektrisch beheizbaren Heizkörpern für eine Verdampfervorrichtung und mit mindestens einer Durchgangsöffnung zum Verdampfen von in der Durchgangsöffnung befindlicher Flüssigkeit umfasst ein Bereitstellen eines plattenförmigen Halbleitermaterials, ein Einarbeiten einer Mehrzahl von sich in Richtung der Flächennormale des Halbleitermaterials durch das Halbleitermaterial erstreckenden Durchgangsöffnungen, ein Trennen des Halbleitermaterials entlang wenigstens einer Trennlinie zur Bereitstellung der Mehrzahl von Heizkörpern.

Die Erfindung hat erkannt, dass eine einem Träger zugewandte Seite des Heizkörpers, der vorteilhaft metallfrei ist und weiter vorteilhaft aus Silizium besteht, typischerweise eine andere Oberflächenbeschaffenheit aufweist als eine dem Heizkörper zugewandte Oberfläche, die das Heizelement kontaktiert. Erfindungsgemäß umfasst das Verfahren ein Anordnen einer der Anzahl von Heizkörpern entsprechenden Anzahl von Dichtungselementen, so dass jede Durchgangsöffnung von einem Dichtungselement umfänglich umschlossen ist, um mit dem erfindungsgemäßen Verfahren Heizkörper für eine flüssigkeitsdichte Verdampfervorrichtung bereitstellen zu können.

Vorteilhaft werden vor dem Trennen des Halbleitermaterials die Dichtungselemente angeordnet, um das Halbleitermaterial zu bearbeiten, bevor durch das Trennen eine Mehrzahl von Heizkörpern entsteht. So kann die Ordnung der herzustellenden Heizkörper, die auf dem Halbleitermaterial exakt angeordnet werden können, genutzt werden. Somit kann eine Mehrzahl von Heizkörpern effektiv hergestellt werden.

Das Anordnen der Dichtungselemente in einem flüssigen Zustand ist bevorzugt, um besonders effektiv die Dichtungselemente auf die Heizkörper aufbringen zu können. Vorteilhaft härten die Dichtungselemente nach dem Anordnen im flüssigen Zustand aus und nehmen einen elastischen, flexiblen beziehungsweise weichen Zustand an.

Vorteilhaft werden die Dichtungselemente beim Anordnen stoffschlüssig mit dem Halbleitermaterial und/oder dem Heizkörper verbunden, um nach dem Anordnen der Dichtungselemente eine so effektiv wie mögliche Anzahl an Bauteilen bereitstellen zu können.

Die Erfindung wird im Folgenden anhand bevorzugter Ausführungsformen unter Bezugnahme auf die beigefügten Figuren erläutert. Dabei zeigt
- Fign. 1 bis 5: je eine Explosionsdarstellung einer Verdampfervorrichtung;
- Fign. 6 bis 9: je eine Querschnittsansicht einer Verdampfervorrichtung;
- Fign. 10 und 11: eine Mehrzahl von Heizkörpern in einer schematischen Draufsicht und einer Querschnittsdarstellung;
- Fig. 12: eine schematische Darstellung eines Inhalators; und
- Fig. 13: eine perspektivische Querschnittsansicht eines Heizkörpers mit einem Flüssigkeitsspeicher.

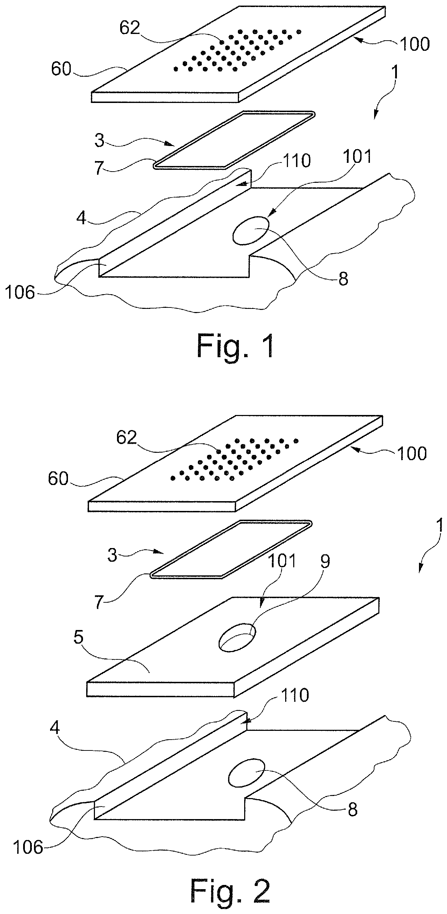

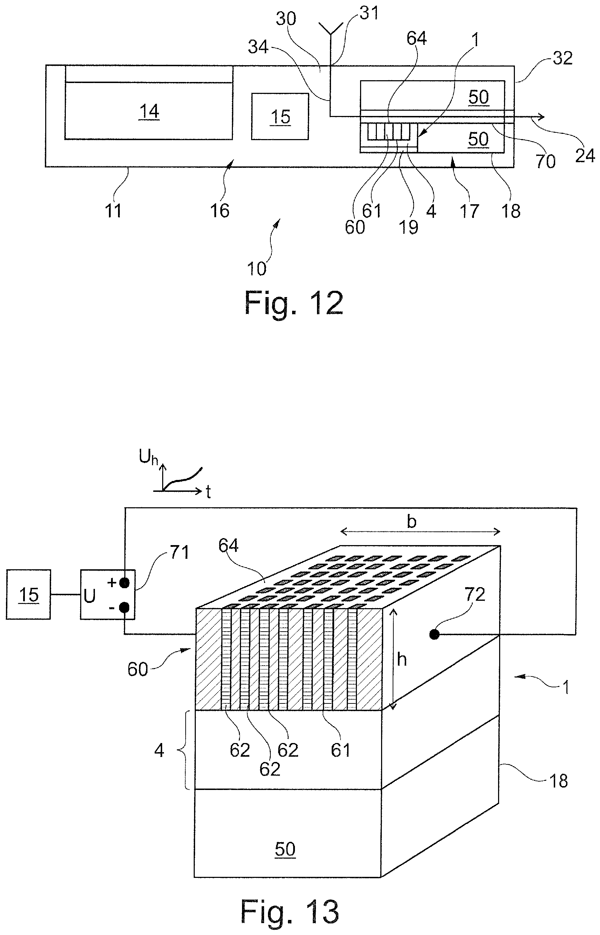

Figuren 1 bis 5 zeigen je eine Explosionsdarstellung einer Verdampfervorrichtung 1. Die Verdampfervorrichtung 1 umfasst einen mit Bezug zu Figur 13 näher erläuterten elektrisch beheizbaren Heizkörper 60 mit mindestens einer Durchgangsöffnung 62 zum Verdampfen von in der Durchgangsöffnung 62 befindlicher Flüssigkeit. In den Ausführungsformen in Figuren 1 bis 5 umfasst die Verdampfervorrichtung 1 einen Träger 4 zur Halterung des Heizkörpers 60. Der Heizkörper 60 ist vom Träger 4 gehalten. Beispielsweise kann der Heizkörper 60 durch Klemmen, Stecken, Kleben, Schweißen und/oder Löten vom Träger 4 gehalten sein.

Der Träger 4 weist vorteilhaft eine Nut bzw. Ausnehmung 106 auf. Die Ausnehmung 106 in dem in Figur 1 gezeigten Beispiel ist so bemessen, dass der Heizkörper 60 im montierten Zustand bündig mit die Ausnehmung 106 definierenden Flanken 110 des Trägers 4 abschließt. Die Anordnung des Heizkörpers 60 in der Ausnehmung 106 kann beispielsweise die Montage der Verdampfervorrichtung 1 begünstigen und die Flüssigkeitsdichtigkeit verbessern.

Der Träger 4 weist wie in Figuren 1 bis 5 gezeigt eine Leitungsöffnung 8 zur Leitung von Flüssigkeit auf. Der Trägers 4 kann mit einem nicht gezeigten Flüssigkeitsspeicher 18 flüssigkeitsleitend verbunden sein. Ein Flüssigkeitsspeicher 18 ist mit Bezug zu Figuren 12 und 13 beschrieben. Der Flüssigkeitsspeicher 18 ist durch die Leitungsöffnung 8 flüssigkeitsleitend mit dem Heizkörper 60 verbunden. In einer bevorzugten Ausführungsform kann sich zwischen dem Flüssigkeitsspeicher 18 und dem Heizkörper 60, beispielsweise in der und/oder durch die Leitungsöffnung 8 eine Dochtstruktur 19 erstrecken, die mit Bezug zu der schematischen Abbildung in Figur 12 erklärt.

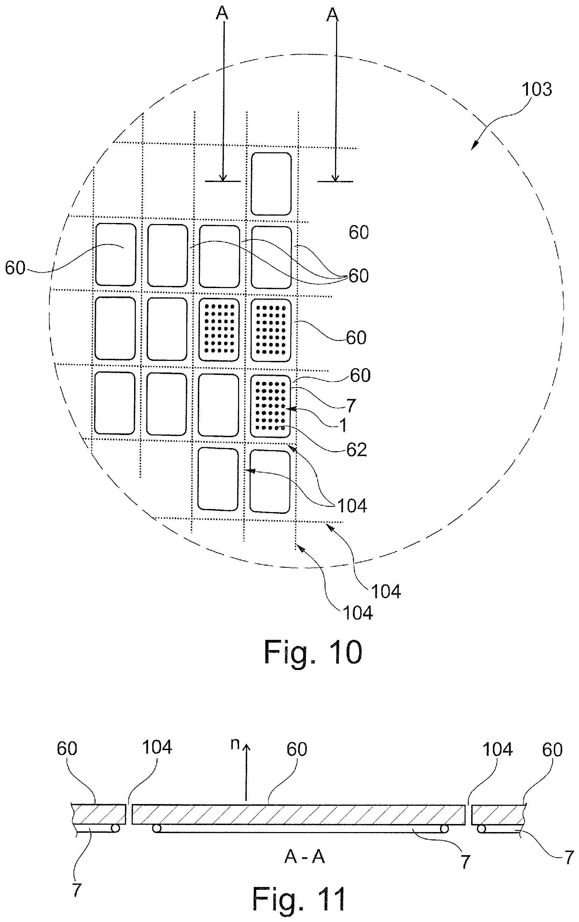

Der Heizkörper 60 weist eine Mehrzahl von Durchgangsöffnungen 62 auf, die in einer vorteilhaften Ausführungsform aufgrund ihrer Abmessungen insbesondere als Mikrokanäle bezeichnet werden können. Die Durchgangsöffnungen 62 sind in diesen Beispielen innerhalb eines Rechtecks angeordnet. Es ist jedoch jede andere Anordnung der Durchgangsöffnungen 62 denkbar. Eine ausführliche Darstellung des Heizkörpers 60 ist in der Beschreibung mit Bezug zu Figur 13 gegeben. Zur Herstellung des Heizkörpers 60 sei auf die Figuren 10 und 11 sowie die dazugehörige Beschreibung verwiesen.

Die Dichtungsvorrichtung 3 umschließt die Durchgangsöffnung 62 dichtend, siehe Figur 1. Die Dichtungsvorrichtung 3 ist zwischen dem Heizkörper 60 und dem Träger 4 angeordnet.

In dem in Figur 1 gezeigten Beispiel bildet das Dichtungselement 7 die Dichtungsvorrichtung 3. Das Dichtungselement 7 umschließt die Durchgangsöffnungen 62 vollumfänglich. Die Form des Dichtungselements 3 ist in diesem Beispiel ein abgerundetes Rechteck und kann in anderen Ausführungsformen rund, oval, eckig oder anders geartet sein, um die Durchgangsöffnung 62 zu umschließen.

Das Dichtungselement 7 ist so angeordnet, dass es eine dem Träger 4 zugewandte Seite 100 des Heizkörpers 60 und eine dem Heizkörper zugewandte Oberfläche 101 des Trägers 4 kontaktiert. Auf diese Weise kann an den Kontaktstellen zwischen der Oberfläche 101 des Trägers 4 und der Seite 100 des Heizkörpers 60 eine mögliche Unebenheit ausgeglichen werden, die sich aus der Oberflächenbeschaffenheit des Heizkörpers 60 und des Trägers 4 bzw. der Seite 100 und der Oberfläche 101 ergibt.

Die Ausführungsform gemäß Figur 2 wird in Hinblick auf die Unterschiede zu Figur 1 erläutert. In der in Figur 2 gezeigten Ausführungsform umfasst die Verdampfervorrichtung 1 ein zwischen dem Träger 4 und dem Heizkörper 60 angeordnetes Substrat 5.

Das Substrat 5 kann der thermischen Entkopplung zwischen dem Heizkörper 60 und dem Träger 4 dienen. Besonders vorteilhaft ist ein Substrat 5, wenn der Träger 4 aus einem Kunststoff besteht. In diesem Fall kann das Substrat 5 vorteilhaft aus einem thermisch stabilen Kunststoff, beispielsweise PEEK, und/oder einem keramischen Stoff gebildet sein. Ein Keramiksubstrat ist inert und temperaturbeständig. Durch Variation der Materialdicke des Substrats 5 kann die thermische Trennung und/oder Abdichtwirkung zwischen dem Träger 4 und Heizkörper 60 angepasst werden.

Das Substrat 5 weist eine Flüssigkeitsöffnung 9 zur Leitung von Flüssigkeit auf. Die Flüssigkeitsöffnung 9 ist vorteilhaft in Deckung mit der Leitungsöffnung 8 angeordnet, um Flüssigkeitstransport zu gewährleisten. Damit ist ein durchgehender Flüssigkeitstransport von einem in dieser Figur nicht gezeigten Flüssigkeitsspeicher 18 durch die Leitungsöffnung 8 und die Flüssigkeitsöffnung 9 zu dem Heizkörper 60 möglich.

In dieser Ausführungsform ist das Dichtungselement 7 so angeordnet, dass das Dichtungselement 7 die dem Träger 4 zugewandte Seite 100 des Heizkörpers 60 und eine dem Heizkörper zugewandte Oberfläche 101 des Substrats 5 kontaktiert. Auf diese Weise kann an den Kontaktstellen zwischen der Oberfläche 101 des Substrats 5 und der Seite 100 des Heizkörpers 60 eine mögliche Unebenheit ausgeglichen werden, die sich aus der Oberflächenbeschaffenheit des Heizkörpers 60 und des Substrats 5 ergibt.

Die Ausnehmung 106 in dem in Figur 2 gezeigten Beispiel ist so bemessen, dass das Substrat 5 und/oder der Heizkörper 60 im montierten Zustand bündig mit die Ausnehmung 106 definierenden Flanken 110 des Trägers 4 abschließt.

In Figuren 1 und 2 ist das Dichtungselement 7 als eine separate Komponente dargestellt, was insbesondere ein standardmäßig erhältlicher und temperaturbeständiger O-Ring sein kann. Auch in anderen Ausführungsformen kann das Dichtungselement 7 ein Standardbauteil sein. Bevorzugt kann das Dichtungselement 7 mit dem Heizkörper 60 stoffschlüssig verbunden sein. Es ist auch möglich ein mit dem Heizkörper 60 stoffschlüssig verbundenes Dichtungselement und ein separates Dichtungselement als Dichtungsvorrichtung 3 vorzusehen. Das Dichtungselement 7 besteht vorteilhaft aus einem temperaturbeständigen Kunststoff, beispielsweise Silikon oder Kautschuk.

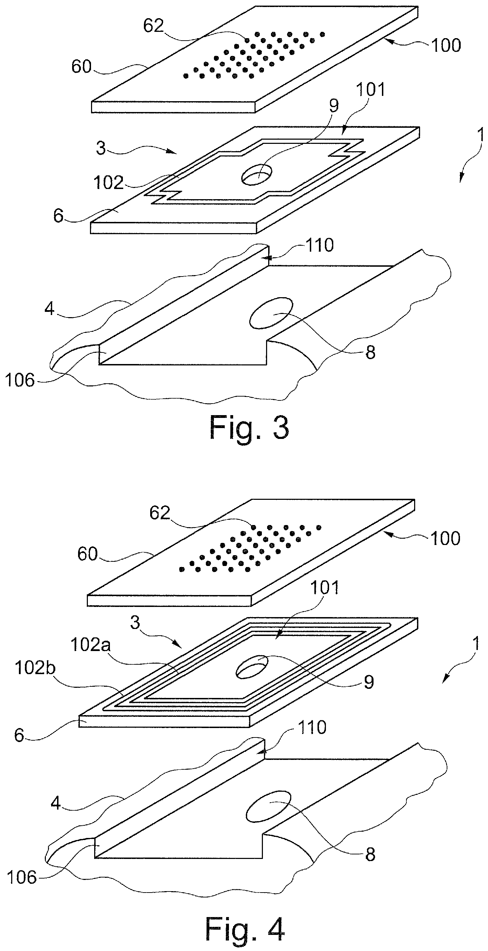

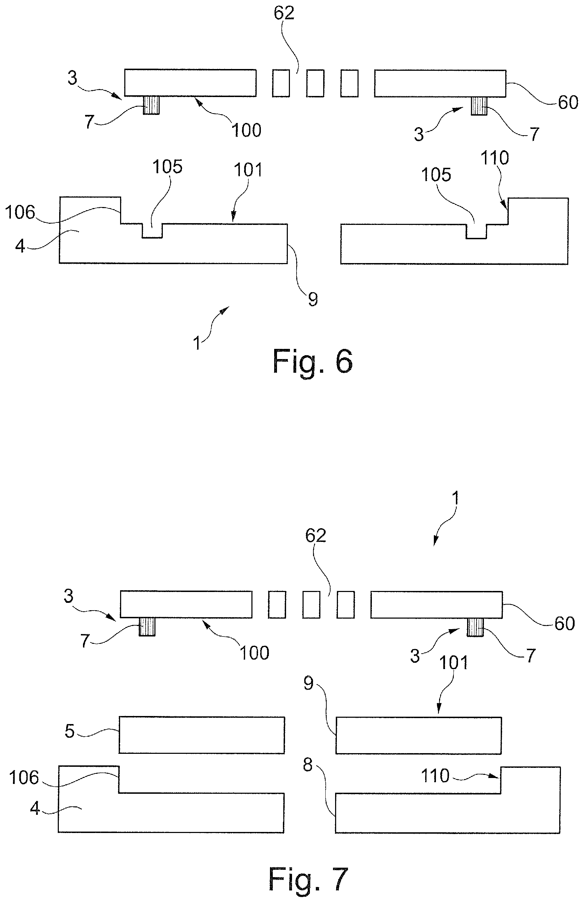

In Figuren 3 und 4 ist je eine Ausführungsform gezeigt, bei der die Verdampfervorrichtung 1 einen zwischen dem Träger 4 und dem Heizkörper 60 angeordneten Dichtungsträger 6 aufweist. Der Dichtungsträger 6 umfasst in Figur 3 ein Dichtelement 102 und in Figur 4 mehrere Dichtelemente 102a, 102b. Die Dichtungsvorrichtung 3 ist in diesen Beispielen durch den Dichtungsträger 6 und/oder das oder die Dichtelemente 102, 102a, 102b gebildet. Das Dichtelement 102 in Figur 3 beziehungsweise die Dichtelement 102a, 102b in Figur 4 können jeweils mit dem Dichtungsträger 6 stoffschlüssig verbunden sein. Es ist auch möglich, dass der Dichtungsträger 106 eine Vertiefung 105 aufweist, die analog zu der in Figur 6 erläuterten Vertiefung 105, beschaffen ist, in die das Dichtelement 102 beziehungsweise die Dichtelemente 102a, 102b eingebracht und gehalten werden können. Dies ist eine Alternative zu der stoffschlüssigen Verbindung zwischen dem Dichtungsträger 6 und dem Dichtelement 102 beziehungsweise den Dichtelementen 102a, 102b.

In dieser Ausführungsform ist das Dichtelement 102 beziehungsweise die Dichtelemente 102a, 102b so angeordnet, dass das Dichtelement 102 beziehungsweise die Dichtelemente 102a, 102b die dem Träger 4 zugewandte Seite 100 des Heizkörpers 60 und eine dem Heizkörper zugewandte Oberfläche 101 des Dichtungsträgers 6 kontaktiert. Auf diese Weise kann an den Kontaktstellen zwischen der Oberfläche 101 des Dichtungsträgers 6 und der Seite 100 des Heizkörpers 60 eine mögliche Unebenheit ausgeglichen werden, die sich aus der Oberflächenbeschaffenheit des Heizkörpers 60 und des Dichtungsträgers 6 ergibt.

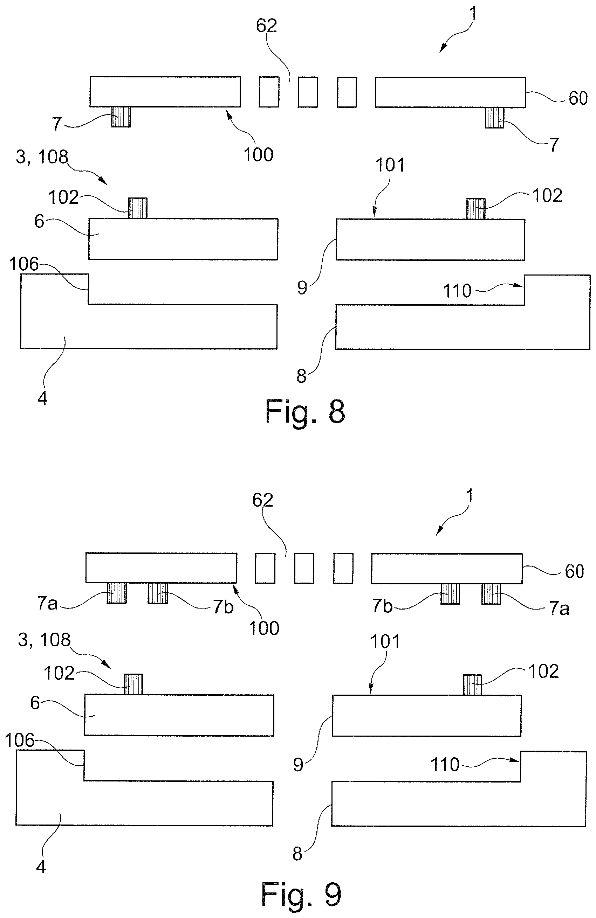

Der in den Figuren 3 und 4 gezeigte Dichtungsträger 6 weist eine mit Bezug zu dem in Figur 2 gezeigten Substrat 5 erklärte Flüssigkeitsöffnung 9 zur Leitung von Flüssigkeit auf. Der Dichtungsträger 6 ist vorteilhaft plattenförmig und kann daher auch als Dichtungsplatte bezeichnet werden. Der Dichtungsträger 6 kann wie das mit Bezug zu Figur 2 beschriebene Substrat 5 bemessen und ausgebildet sein. Der Dichtungsträger 6 wirkt vorteilhaft durch beispielsweise eine geeignete Wahl eines Materials und/oder der Geometrie thermisch isolierend, siehe auch Figuren 8 und 9 und deren Beschreibung.

In Figur 4 sind die Dichtelemente 102a, 102b konzentrisch um die Flüssigkeitsöffnung 9 angeordnet. Somit umschließen die Dichtelemente 102a, 102b die Durchgangsöffnungen 62 vollumfänglich und flüssigkeitsdicht. Die Dichtelemente 102a, 102b sind voneinander ausgehend von der Flüssigkeitsöffnung 9 radial beabstandet. In anderen Ausführungsformen kann beispielsweise eines der Dichtelemente 102a, 102b die Durchgangsöffnungen 62 nur teilweise, insbesondere in besonders exponierten Bereichen, umschließen, wobei besonders exponierte Bereiche beispielsweise die Bereiche sind, in denen eine Leckage wahrscheinlich ist.

Die Ausnehmung 106 des Träger 4 ist so bemessen, dass der Dichtungsträger 6 und/oder der Heizkörper 60 im montierten Zustand bündig mit die Ausnehmung 106 definierenden Flanken 110 des Trägers 4 abschließt.

Das Dichtelement 102 beziehungsweise die Dichtelemente 102a, 102b bestehen vorteilhaft aus einem temperaturbeständigen Kunststoff, beispielsweise Silikon oder Kautschuk.

Zusätzlich zu dem Dichtelement 102 und/oder den Dichtelementen 102a, 102b kann ein Dichtungselement 3 vorgesehen sein, wie mit Bezug zu Figuren 8 und 9 erläutert.

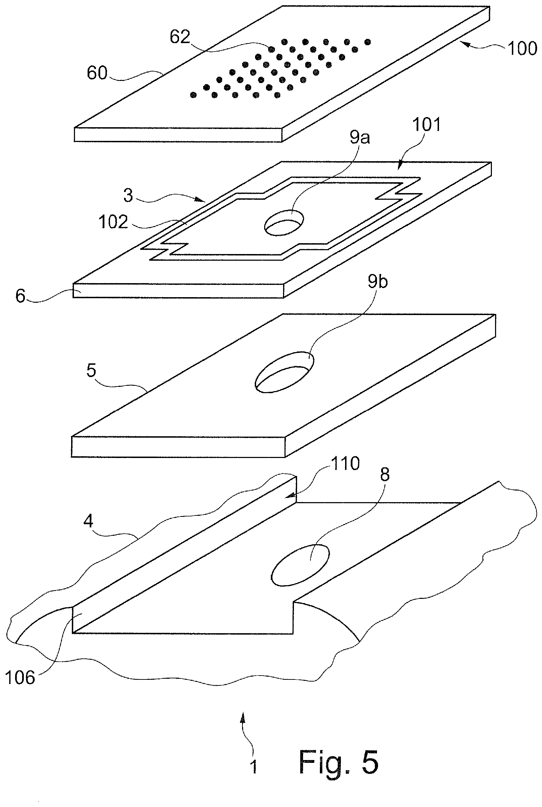

In Figur 5 ist eine Ausführungsform gezeigt, bei der die Verdampfervorrichtung 1 einen zwischen dem Träger 4 und dem Heizkörper 60 angeordnetes Substrat 5, wie mit Bezug zu Figur 2 erläutert, aufweist und einen zwischen dem Träger 4 und dem Heizkörper 60 angeordneten Dichtungsträger 6, wie mit Bezug zu Figuren 3 und 4 erläutert. Hier ist der Dichtungsträger 6 zwischen dem Heizkörper 60 und dem Substrat 5 angeordnet. Das Substrat 5 und der Dichtungsträger 6 weisen je eine mit Bezug zu dem in Figur 2 gezeigten Substrat 5 erklärte Flüssigkeitsöffnung 9a, 9b zur Leitung von Flüssigkeit auf.

Die Ausnehmung 106 in dem in Figuren 5 gezeigten Beispielen ist so bemessen, dass das Substrat 5, der Dichtungsträger 6 und/oder der Heizkörper 60 im montierten Zustand bündig mit die Ausnehmung 106 definierenden Flanken 110 des Trägers 4 abschließt.

Durch die vorteilhaft polierte, insbesondere einseitig polierte, single side polished (ssp), Oberfläche beziehungsweise Seite 100 des Heizkörpers 60 und einer form- und lagegenauen Oberfläche 101 des Substrats 5 sowie des Trägers 4 kann die Abdichtung der Bauteile, insbesondere der Verdampfervorrichtung 1, verbessert werden. Vorteilhaft kann ein- oder beidseitig das Dichtelement 102, oder die Dichtelemente 102a, 102b auf den Dichtungsträger 6 und/oder ein oder mehrere Dichtungselemente 7 auf den Heizkörper 60 aufgebracht werden.

Figuren 6 bis 9 zeigen je eine schematische Querschnittsansicht einer Verdampfervorrichtung 1.

In Figur 6 weist die Dichtungsvorrichtung 3 ein Dichtungselement 7 auf. Eine das Dichtungselement 7 kontaktierende Oberfläche 101 weist eine der Geometrie des Dichtungselements 7 entsprechende Vertiefung 105 zum Einfügen des Dichtungselements 7 auf.

Das Dichtungselemente 7 kann eine Höhe aufweisen, die der Tiefe der Vertiefung 105 entspricht. Vorteilhaft weist das Dichtungselement 7 eine der Breite der Vertiefung 105 entsprechende Breite auf. Somit kann das Dichtungselement 7 die Vertiefung flächig kontaktieren. Durch das Einfügen des Dichtungselements 7 in die Vertiefung 105 ist diese Ausführungsform besonders flüssigkeitsdicht und kompakt.

Die Ausnehmung 106 wird durch als Flanken 110 bezeichnete Oberflächen definiert. Die Flanken 110 können eine Höhe aufweisen, die der Dicke des Heizkörpers 60 entspricht. Der Heizkörper 60 lässt sich somit bündig abschließend in die Ausnehmung 106 einfügen. Damit weist die Verdampfervorrichtung 1 im montierten Zustand eine bündige Oberseite auf, die durch den Heizkörper 60 und den Träger 4 definiert ist. Die Tiefe der Ausnehmung 106 kann an die Höhe des Heizkörpers 60, analog an die Höhe des Heizkörpers 60 und einem Substrat 5, des Heizkörpers 60 und einem Dichtungsträger 6 und/oder des Heizkörpers 60 und einem Substrat 5 und einem Dichtungsträger 6 angepasst werden.

Figur 7 ist ein Querschnitt der mit Bezug zu Figur 2 erläuterten Ausführungsform mit einem zwischen dem Heizkörper 60 und dem Träger 4 angeordneten Substrat 5. Diese Ausführungsform ist besonders für die thermische Entkopplung von Heizkörper 60 und Träger 4 geeignet. Das Substrat 5 und die Dichtungselemente 7 hemmen jeweils den Wärmetransport vom Heizkörper 60 zum Träger 4.

Figur 8 ist ein Querschnitt der mit Bezug zu Figur 3 erläuterten Ausführungsform mit einem zwischen dem Heizkörper 60 und dem Träger 4 angeordneten Dichtungsträger 6 mit einem Dichtelement 102. Die Dichtungsvorrichtung 3 umfasst ein Dichtungselement 7 und den Dichtungsträger 6 mit dem Dichtelement 102. Die Dichtungsvorrichtung 3 bildet im montierten Zustand eine einfache Ausführungsform einer Labyrinthdichtung 108. Diese Ausführungsform ist besonders flüssigkeitsdicht und entkoppelt den Heizkörper 60 und den Träger thermisch besonders effektiv.

Die Ausführungsform gemäß Figur 9 unterscheidet sich zu der in Figuren 3 und 8 gezeigten Ausführungsform dadurch, dass zwischen dem Heizkörper 60 und dem Dichtungsträger 6 mehrere, hier zwei, Dichtungselemente 7a, 7b angeordnet sind. Die Dichtungsvorrichtung 3 umfasst zwei Dichtungselemente 7a, 7b und den Dichtungsträger 6 mit dem Dichtelement 102. Die Dichtungsvorrichtung 3 bildet im montierten Zustand eine Labyrinthdichtung 108. Diese Ausführungsform ist besonders flüssigkeitsdicht und entkoppelt den Heizkörper 60 und den Träger thermisch besonders effektiv.

In Figur 9 sind die Dichtungselemente 7a, 7b konzentrisch um die Durchgangsöffnungen 62 angeordnet. Somit umschließen die Dichtungselemente 7a, 7b die Durchgangsöffnungen 62 vollumfänglich und flüssigkeitsdicht. Die Dichtungselemente 7a, 7b sind voneinander ausgehend von den Durchgangsöffnungen 62 radial beabstandet. In anderen Ausführungsformen kann beispielsweise eines der Dichtungselemente 7a, 7b die Durchgangsöffnungen 62 nur teilweise, insbesondere in besonders exponierten Bereichen, umschließen, in denen eine Leckage wahrscheinlich ist. Zusätzlich kann eines der Dichtungselemente 7a, 7b beispielsweise im Randbereich der Durchgangsöffnung 62 auch zum Beispiel labyrinthartig ausgeformt sein, um eine zusätzliche fluidische Abdichtwirkung gegen ein unbeabsichtigt austretenden Fluid, z.B. durch Druckschwankungen, wie kurzzeitigen Überdruck, zu bewerkstelligen.

In den Ausführungsform gemäß Figuren 8 und 9 können auf dem Dichtungsträger 6 auch mehrere Dichtelemente 102a, 102b angeordnet sein (nicht in den Figuren gezeigt). Die zuvor erläuterte Labyrinthdichtung 108 und/oder das Einfügen eines Dichtungselements 7 in eine Vertiefung 105 kann als Positionier- beziehungsweise Montagehilfe und/oder zusätzliche mechanische Fixierung des Heizkörpers 60 am Träger 4 genutzt werden.

Figur 10 zeigt eine Mehrzahl von Heizkörpern 60 in einer schematischen Draufsicht und Figur 11 zeigt die Mehrzahl von Heizkörpern 60 in einer Querschnittsdarstellung in einer Ebene, durch die in Figur 10 dargestellte Linie A verläuft.

Die Heizkörper 60 liegen als Halbleiterchip vor, die jeweils in ihrem mittleren Bereich ein Feld mit einer Vielzahl von Durchgangsöffnungen 62 aufweisen, mit denen die Heizkörper 60 flüssigkeitsdurchlässig durchlöchert sind. Die Durchgangsöffnungen 62 sind in Figur 10 nur bei einigen Heizkörpern 60 als Punkte angedeutet, werden jedoch mit Bezug zu Figur 13 näher erläutert.

Anhand von Figuren 10 und 11 wird das erfindungsgemäße Verfahren zur Herstellung einer Mehrzahl von elektrisch beheizbaren Heizkörpern 60 mit mindestens einer Durchgangsöffnung 62 zum Verdampfen von in der Durchgangsöffnung 62 befindlicher Flüssigkeit erläutert.

Es wird ein plattenförmiges Halbleitermaterial 103 bereitgestellt. Das Halbleitermaterial 103 ist vorteilhaft metallfrei, beispielsweise aus Silizium, um eine schädliche Abgabe von Metallbestandteilen und/oder Metallatomen oder -ionen oder eine schädliche katalytische Wirkung von metallischen Bestandteilen beim Verdampfen durch den Heizkörper 60 zu vermeiden.

Das plattenförmiges Halbleitermaterial 103 ist im Wesentlich eben und weist eine in Figur 11 angedeutete wohldefinierte Flächennormale n auf, die senkrecht auf dem plattenförmigen Halbleitermaterial 103 steht. Das plattenförmige Halbleitermaterial 103 liegt in Form eines Wafers mit einem Durchmesser von beispielsweise 8 Zoll vor und weist zwei gegenüberliegende Seiten auf, die als Oberseite und Unterseite bezeichnet werden können.

Auf wenigstens eine der beiden Seiten kann wenigstens eine Isolierschicht aufgebracht werden, beispielsweise aus Siliziumnitrid und/oder Siliziumoxinitrid. Eine andere oder weitere Isolierschicht kann aus thermischem Oxid gebildet sein. Beispielsweise kann die Isolierung durch Gasabscheidung aufgebracht werden.

Auf das Halbleitermaterial 103 kann Polysilizium, beispielsweise ebenfalls durch Gasabscheidung, aufgebracht werden, um die zu fertigenden Heizkörper 60 mit einer heißleitenden Schicht (NTC-Schicht) zu versehen.

In das Halbleitermaterial 103 wird eine Mehrzahl von sich in Richtung der Flächennormale durch das Halbleitermaterial 103 erstreckenden Durchgangsöffnungen 62 eingearbeitet, beispielsweise durch Fotolithographie.

Das Halbleitermaterials 103 wird entlang einer oder mehrerer Trennlinien 104 zur Bereitstellung der Mehrzahl von Heizkörpern 60 getrennt. Dieser als "dicing" bezeichnete Schritt trennt das Halbleitermaterial 103 in einzelne Heizkörper 60 oder Gruppen von Heizkörpern 60. Es können aus dem Halbleitermaterial 103 beispielsweise in Form eines 8"-Wafers zwischen 1000 und 10000, vorzugsweise zwischen 2000 und 6000, beispielsweise etwa 4200 Heizkörper 60 ausgeschnitten werden.

Erfindungsgemäß wird eine wenigstens der Anzahl von Heizkörpern 60 entsprechende Anzahl von Dichtungselementen 7 so angeordnet, dass jede Durchgangsöffnung 62 von einem Dichtungselement 7 umfänglich umschlossen ist. Somit kann bei der Fertigung eines Heizkörpers 60 eine spätere Leckage in Richtung der Liquidzuführung einer Verdampfervorrichtung 1 umfassend den Heizkörper 60 vermieden werden. Dafür werden umlaufend um die Konturen der jeweiligen Durchgangsöffnungen 62 Dichtungsbahnen beziehungsweise Dichtungselemente 7 aufgebracht.

Jeder Heizkörper 60 wird so beispielsweise mit einem vorteilhaft thermischen stabilen Kunststoff belegt. Beispielsweise weist jeder Heizkörper 60 nach der Herstellung ein Dichtungselement 7 auf.

Damit weist eine mit Bezug zu Figuren 1 bis 9 erläuterte Dichtungsvorrichtung 3 ein Dichtungselement 7 auf, wobei das Dichtungselement 7 im montierten Zustand die Durchgangsöffnung 62 dichtend umschließt und die Dichtungsvorrichtung 3 ist bei sachgerechter Montage zwischen dem Heizkörper 60 und einem Träger 4 einer Verdampfervorrichtung 1 angeordnet.

Vorteilhaft werden vordem Trennen des Halbleitermaterials 103 die Dichtungselemente 7 um die Durchgangsöffnungen 62 der jeweiligen Heizkörper 60 angeordnet beziehungsweise aufgebracht, um Heizkörper 60 möglichst effektiv herstellen zu können. Die Dichtungselemente 7 werden dabei schablonenartig auf das Halbleitermaterial 103 aufgebracht. In anderen Ausführungsformen kann das Aufbringen der Dichtungselemente 7 auch nach dem Trennen des Halbleitermaterials 103 erfolgen, um die Qualität der Dichtungselemente 7 nicht durch das Trennen zu gefährden.

Die Dichtungselemente 7 bestehen vorteilhaft aus temperaturbeständigem Silikon und/oder Kautschuk. Das Material des Dichtungselements 7 ist vorteilhaft so gewählt, dass es temperaturbeständig und nach der Herstellung hinreichend inert ist. Die aufgetragene Dichtungsbahn beziehungsweise das aufgetragene Dichtungselement 7 kann durch Variation der Geometrie, d.h. Dicke, Breite, Form, Aufdruckmuster, an die Anforderungen an die Flüssigkeitsdichtigkeit und die thermische Entkopplung zwischen den Heizkörper 60 und einem Träger 4 angepasst werden.

Für die Anordnung beziehungsweise geometrische Ausprägung der Dichtungselemente 7 ist es vorteilhaft, sowohl den Heizkörper 60 als auch das Dichtungselement 7 optimal zu gestalten, beispielsweise indem funktionale Bereiche auf den Heizkörpern 60 vorgesehen sind, die von dem Dichtungselement 7 ausgespart werden.

Das Anordnen der Dichtungselemente 7 kann vorteilhaft in einem flüssigen Zustand, beispielsweise durch Spritzen, Spritzgießen, Sprühen, 3D-Druck, Gießen und/oder das Auftragen einer Schweißraupe erfolgen. Das Anordnen der Dichtungselemente 7 kann auch durch Aufdruckverfahren, beispielsweise Tampondruck oder Siebdruckverfahren, auf den Wafer beziehungsweise das Halbleitermaterial 103 aufgebracht werden.

Die Dichtungselemente 7 werden vorteilhaft beim Anordnen stoffschlüssig mit dem Halbleitermaterial 103 und/oder dem Heizkörper 60 verbunden.

Nach dem Aufbringen beziehungsweise Applizieren härtet das Dichtungselement 7 vorteilhaft aus, wobei das Dichtungselement 7 nach dem Aushärten vorteilhaft weicher als das Halbleitermaterial 103 ist, d.h. das Dichtungselement 7 nimmt beim Aushärten nach dem Applizieren eine definierte Elastizität an, die weicher als die des Halbleitermaterials 103 ist.

Figur 12 zeigt schematisch einen Inhalator 10. Der Inhalator 10, hier ein elektronisches Zigarettenprodukt, umfasst ein Gehäuse 11, in dem ein Luftkanal 30 zwischen mindestens einer Lufteinlassöffnung 31 und einer Luftauslassöffnung 24 an einem Mundende 32 des Zigarettenprodukts 10 vorgesehen ist. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und einen Luftstrom 34 in dem Luftkanal 30 erzeugt.

Das Zigarettenprodukt 10 besteht vorteilhaft aus einem Basisteil 16 und einer Verbrauchseinheit 17, die die Verdampfervorrichtung 1 und den Flüssigkeitsspeicher 18 umfasst und insbesondere in Form einer auswechselbaren Kartusche ausgebildet ist. Die durch die Einlassöffnung 31 angesaugte Luft wird in dem Luftkanal 30 zu der, oder durch die mindestens eine Verdampfervorrichtung 1 geleitet. Die Verdampfervorrichtung 1 ist mit dem Flüssigkeitsspeicher 18 verbunden oder verbindbar, in dem mindestens eine Flüssigkeit 50 gespeichert ist.

Die Verdampfervorrichtung 1 verdampft Flüssigkeit 50, die der Verdampfervorrichtung 1 aus dem Flüssigkeitsspeicher 18 vorteilhaft von einem Docht beziehungsweise einer Dochtstruktur 19 mittels Kapillarkräften zugeführt wird, und gibt die verdampfte Flüssigkeit als Aerosol/Dampf an einer Auslassseite 64 in den Luftstrom 34 zu.

An einer Einlassseite 61 des Heizkörpers 60 ist vorteilhaft die poröse und/oder kapillare, flüssigkeitsleitende Dochtstruktur 19 angeordnet, wie schematisch in Figur 12 gezeigt. Die in Figur 12 gezeigte Anbindung der Dochtstruktur 19 an den Flüssigkeitsspeicher 18 und an den Heizkörper 60 über den Träger 4 ist nur beispielhaft zu verstehen. Insbesondere können eine Flüssigkeitsschnittstelle und/oder mehrere Flüssigkeitsleitungen zwischen Flüssigkeitsspeieher 18 und Dochtstruktur 19 vorgesehen sein. Der Flüssigkeitsspeicher 18 kann daher auch beabstandet von der Dochtstruktur 19 angeordnet sein. Die Dochtstruktur 19 kontaktiert die Einlassseite 61 des Heizkörpers 60 vorteilhaft flächig und deckt sämtliche Durchgangsöffnungen 62 einlassseitig ab. An der dem Heizkörper 60 gegenüberliegenden Seite ist die Dochtstruktur flüssigkeitsleitend mit dem Flüssigkeitsspeicher 18 verbunden. Der Flüssigkeitsspeicher 18 kann in seinen Abmessungen größer als die Dochtstruktur 19 sein. Die Dochtstruktur 19 kann beispielsweise in eine Öffnung eines Gehäuses des Flüssigkeitsspeichers 18 eingesetzt sein. Es kann auch eine Mehrzahl von Verdampfervorrichtungen 1 einem Flüssigkeitsspeicher 18 zugeordnet sein. Die Dochtstruktur 19 kann generell einteilig oder mehrteilig sein.

Die Dochtstruktur 19 besteht aus porösem und/oder kapillarem Material, das aufgrund von Kapillarkräften in der Lage ist, von dem Heizkörper 60 verdampfte Flüssigkeit in ausreichender Menge von dem Flüssigkeitsspeicher 18 zu dem Heizkörper 60 passiv nachzufördern, um ein Leerlaufen der Durchgangsöffnungen 62 und sich daraus ergebende Probleme zu verhindern.

Die Dochtstruktur 19 besteht vorteilhaft aus einem elektrisch nichtleitenden Material, um eine unerwünschte Erwärmung von Flüssigkeit in der Dochtstruktur 19 durch Stromfluss zu vermeiden. Die Dochtstruktur 19 weist vorteilhaft eine geringe thermische Leitfähigkeit auf. Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitze-beständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm3 und 10 mm3, weiter vorzugsweise im Bereich zwischen 2 mm3 und 8 mm3, noch weiter vorzugsweise im Bereich zwischen 3 mm3 und 7 mm3 und beträgt beispielsweise 5 mm3.

Falls die Dochtstruktur 19 aus einem elektrisch und/oder thermisch leitenden Material besteht, was nicht ausgeschlossen ist, ist zwischen der Dochtstruktur 19 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Durchgangsöffnungen 62 korrespondierenden Öffnungen vorgesehen.

Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugs-weise zwischen 0,7 ml und 2 ml oder 1,5 ml.

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-Ionen-Akku, sein. Die Verbrauchseinheit 17 ist zwischen dem Energiespeicher 14 und dem Mundende 32 angeordnet. Die elektronische Steuerungsvorrichtung 15 umfasst mindestens eine digitale Datenverarbeitungseinrichtung, insbesondere Mikroprozessor und/oder Microcontroller, in dem Basisteil 16 (wie in Figur 12 gezeigt) und/oder in der Verbrauchseinheit 17.

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Zigarettenprodukts 10 zieht, um zu inhalieren. In diesem Fall steuert die Steuerungsvorrichtung 15 die Verdampfervorrichtung 1 an, um Flüssigkeit 50 aus dem Flüssigkeitsspeicher 18 als Aerosol/Dampf in den Luftstrom 34 zuzugeben.

Die Verdampfervorrichtung 1 beziehungsweise der mindestens eine Verdampfer 60 ist in einem dem Mundende 32 abgewandten Teil der Verbrauchseinheit 17 angeordnet. Damit ist eine effektive elektrische Kopplung und Ansteuerung der Verdampfervorrichtung 1 möglich. Der Luftstrom 34 führt vorteilhaft durch einen axial durch den Flüssigkeitsspeicher 18 laufenden Luftkanal 70 zu der Luftauslassöffnung 24.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff insbesondere Nikotin. Die angegebenen Bestandteile der Flüssigkeit 50 sind jedoch nicht zwingend. Insbesondere kann auf Aroma- und/oder Wirkstoffe, insbesondere Nikotin, verzichtet werden.

Die Verbrauchseinheit bzw. Kartusche 17 oder das Basisteil 16 umfasst vorteilhaft einen nichtflüchtigen Datenspeicher zum Speichern von die Verbrauchseinheit bzw. Kartusche 17 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft elektrisch mit der Steuereinrichtung 15 verbunden oder verbindbar.

In dem Inhalator 10 und/oder in einem externen Speicher, der in geeigneter und an sich bekannter Weise, zumindest zeitweilig, kommunikationstechnisch mit dem Inhalator 10 verbunden werden kann, könnten auch nutzerbezogene Daten, insbesondere über das Rauchverhalten, gespeichert und vorzugsweise auch zur Steuerung und Regelung des Inhalators genutzt werden.

In Figur 13 ist eine Verdampfervorrichtung 1 gezeigt. Die Verdampfervorrichtung 1 umfasst einen blockförmigen, vorzugsweise monolithischer Heizkörper 60 vorzugsweise aus einem elektrisch leitenden Material, insbesondere einem Halbleitermaterial vorzugsweise Silizium, und einen Träger 4. Es ist nicht erforderlich, dass der gesamte Heizkörper 60 aus einem elektrisch leitenden Material besteht. Es kann beispielsweise ausreichen, dass die Oberfläche des Heizkörpers 60 elektrisch leitend, beispielsweise metallisch, beschichtet oder vorzugsweise geeignet dotiert ist. In diesem Fall muss nicht die gesamte Oberfläche beschichtet sein, beispielsweise können metallische oder vorzugsweise nichtmetallische oder nichtmetallisch kaschierte matallische Leiterbahnen auf einem nichtleitenden beziehungsweise halbleitenden Grundkörper vorgesehen sein. Es ist auch nicht zwingend erforderlich, dass der gesamte Heizkörper 60 heizt; es kann beispielsweise ausreichen, wenn ein Abschnitt oder eine Heizschicht des Heizkörpers 60 im Bereich der Austrittsseite 64 heizt.

Der Heizkörper 60 ist mit einer Mehrzahl von Mikrokanälen beziehungsweise Durchgangsöffnungen 62 versehen, die eine Einlassseite 61 des Heizkörper 60 mit einer Auslassseite 64 des Heizkörper 60 flüssigkeitsleitend verbinden. Die Einlassseite 61 ist über eine nicht in Figur 13 gezeigte Dochtstruktur 19 flüssigkeitsleitend mit dem Flüssigkeitsspeicher 18 verbunden. Die Dochtstruktur 19 dient zur passiven Förderung von Flüssigkeit aus dem Flüssigkeitsspeicher 18 zum Heizkörper 60 mittels Kapillarkräften.

Der mittlere Durchmesser der Durchgangsöffnungen 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in eine Durchgangsöffnung 62 eindringende Flüssigkeit durch die Durchgangsöffnung 62 nach oben steigt, bis die Durchgangsöffnung 62 mit Flüssigkeit gefüllt ist. Das Volumenverhältnis von Durchgangsöffnungen 62 zu Heizkörper 60, das als Porosität des Heizkörper 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Durchgangsöffnungen 62 versehenen Flächen des Heizkörper 60 liegen beispielsweise im Bereich zwischen 0,5 mm und 3 mm, vorzugsweise zwischen 0.5 mm und 1 mm. Die Abmessungen der mit Durchgangsöffnungen 62 versehenen Flächen des Heizkörpers 60 können beispielsweise betragen: 0,95 mm x 1,75 mm oder 1,9 mm x 1,75 mm oder 1,9 mm x 0,75 mm. Die Kantenlängen des Heizkörpers 60 können beispielsweise im Bereich zwischen 0,5 mm und 5 mm, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm x 3 mm, 2mm x 2 mm oder 2 mm x 3 mm betragen.

Die Breite b des Heizkörper 60 (siehe Figur 13) liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörper 60 (siehe Figur 13) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm. Auch noch kleinere Heizkörper 60 können gefertigt, vorgesehen und funktionsgerecht betrieben werden.

Die Anzahl der Durchgangsöffnungen 62 liegt vorzugsweise im Bereich zwischen vier und 1000. Auf diese Weise lässt sich der Wärmeeintrag in die Durchgangsöffnungen 62 optimieren und eine gesicherte hohe Verdampfungsleistung sowie eine ausreichend große Dampfaustrittsfläche realisieren.

Die Durchgangsöffnungen 62 sind in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Durchgangsöffnungen 62 mit gesichert hoher Verdampfungsleistung realisieren.

Der Querschnitt der Durchgangsöffnungen 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge einer oder jeder Durchgangsöffnung 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von Heizkörper 60 in die Durchgangsöffnungen 62 realisieren.

Der Abstand zweier Durchgangsöffnungen 62 beträgt vorzugsweise mindestens das 1,3-fache des lichten Durchmessers einer Durchgangsöffnung 62, wobei der Abstand auf die Mittelachsen der beiden Durchgangsöffnungen 62 bezogen ist. Der Abstand kann bevorzugt das 1,5- bis 5-fache, weiter bevorzugt das 2- bis 4-fache des lichten Durchmessers einer Durchgangsöffnung 62 betragen. Auf diese Weise lässt sich ein optimaler Wärmeeintrag in den Heizkörper 60 und eine ausreichend stabile Anordnung und Wandstärke der Durchgangsöffnungen 62 realisieren.

Aufgrund der vorbeschriebenen Merkmale kann der Heizkörper 60 auch als Volumenheizer bezeichnet werden.

Die Verdampfervorrichtung 1 weist eine vorzugsweise von der Steuerungsvorrichtung 29 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohm'schen Widerstands des elektrisch leitenden Heizkörpers 60 führt der Stromfluss zu einer Erhitzung des Heizkörpers 60 und daher zu einer Verdampfung von in den Durchgangsöffnungen 62 enthaltener Flüssigkeit. Auf diese Weise erzeugter Dampf/Aerosol 6 entweicht zur Auslassseite 64 aus den Durchgangsöffnungen 62 und wird dem Luftstrom 34 beigemischt, siehe Figur 12. Genauer steuert bei Feststellung eines durch Ziehen des Konsumenten verursachten Luftstroms 34 durch den Luftkanal 30 die Steuerungsvorrichtung 29 die Heizspannungsquelle 71 an, wobei durch spontane Erhitzung die in den Durchgangsöffnungen 62 befindliche Flüssigkeit in Form von Dampf/Aerosol 6 aus den Durchgangsöffnungen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Eine elektronische beziehungsweise elektrische Anbindung des Heizkörpers 60 kann beispielsweise über Klemm-, Feder- oder Presskontakte, wirebonding und/oder Löten erfolgen.

Vorzugsweise ist in dem Datenspeicher des Inhalators 10 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Durchgangsöffnungen 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100 °C und 400 °C, weiter bevorzugt zwischen 150 °C und 350 °C, noch weiter bevorzugt zwischen 190 °C und 290 °C.

Der Heizkörper 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise 750 µm, noch weiter vorzugsweise kleiner oder gleich 500 µm aufweist. Oberflächen des Heizkörpers 60 können vorteilhaft hydrophil sein. Die Auslassseite 64 des Heizkörpers 60 kann vorteilhaft mikrostrukturiert sein bzw. Mikroausnehmungen (micro grooves) aufweisen.

Die Verdampfervorrichtung 1 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfervorrichtung 1 hinsichtlich der Flüssigkeits-/Dampfmenge pro Zug, d.h. je Zugdauer von 1 s bis 3 s, einstellbar sein.

Im Folgenden wird beispielhaft der Ablauf des Verdampfungsvorgangs erläutert.

In einem Ausgangszustand ist die Spannungsquelle 71 beziehungsweise der Energiespeicher 14 für den Heizvorgang ausgeschaltet.

Zum Verdampfen von Flüssigkeit 50 wird die Spannungsquelle 14, 71 für den Heizkörper 60 aktiviert. Die Spannung Uh wird dabei so eingestellt, dass die Verdampfungstemperatur in dem Heizkörper 60 und somit in den Durchgangsöffnungen 62 an das individuelle Verdampfungsverhalten des eingesetzten Flüssigkeitsgemischs angepasst ist. Dies verhindert die Gefahr von lokaler Überhitzung und dadurch Schadstoffentstehung.

Insbesondere kann auch einer unerwünschten differentiellen Verdampfung eines Liquidgemisches entgegengewirkt oder begegnet werden oder eine solche vermieden werden. Ein Liquidgemisch könnte sonst Komponenten aufgrund unterschiedlicher Siedetemperaturen vorschnell im Laufe einer Abfolge von Verdampfungsvorgängen, insbesondere "puffs", verlieren, bevor das Reservoir 18 des Liquids 50 vollständig entleert ist, was beim Betrieb unerwünschte Effekte wie beispielsweise die mangelnde Konstanz der Dosierung bei einem Benutzer nach sich ziehen könnte, insbesondere bei einem pharmazeutisch wirksamen Liquid.

Sobald eine Flüssigkeitsmenge verdampft ist, die dem Volumen der Durchgangsöffnungen 62 entspricht oder damit in Zusammenhang steht, wird die Heizspannungsquelle 71 deaktiviert. Da die Liquideigenschaften und -menge vorteilhaft exakt bekannt sind und der Heizkörper 60 einen messbaren temperaturabhängigen Widerstand aufweist, kann dieser Zeitpunkt sehr genau bestimmt bzw. gesteuert werden. Die Energieaufnahme der Verdampfervorrichtung 1 lässt sich daher gegenüber bekannten Vorrichtungen reduzieren, da die benötigte Verdampfungsenergie dosierter und damit exakter eingebracht werden kann.

Nach Abschluss des Heizvorgangs sind die Durchgangsöffnungen 62 überwiegend oder vollständig entleert. Die Heizspannung 71 wird dann so lange ausgeschaltet gehalten, bis mittels Nachförderung von Flüssigkeit durch die Dochtstruktur 19 die Durchgangsöffnungen 62 wieder aufgefüllt sind. Sobald dies der Fall ist, kann der nächste Heizzyklus durch einschalten der Heizspannung 71 begonnen werden.

Die von der Heizspannungsquelle 71 erzeugte Ansteuerfrequenz des Heizkörpers 60 liegt im Allgemeinen vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Die Frequenz und der Tastgrad der Heizspannung Uh für den Heizkörper 60 sind vorteilhaft an die Eigenschwingung bzw. Eigenfrequenz der Blasenschwingungen während der Blasensiedung angepasst. Vorteilhaft kann die Periodendauer 1/f der Heizspannung daher im Bereich zwischen 5 ms und 50 ms, weiter vorteilhaft zwischen 10 ms und 40 ms, noch weiter vorteilhaft zwischen 15 ms und 30 ms liegen und beispielsweise 20 ms betragen. Je nach Zusammensetzung der verdampften Flüssigkeit 50 können andere als die genannten Frequenzen optimal an die Eigenschwingung bzw. Eigenfrequenz der Blasenschwingungen angepasst sein.

Des Weiteren hat sich gezeigt, dass der durch die Heizspannung Uh erzeugten maximale Heizstrom vorzugsweise nicht mehr als 7 A, weiter vorzugsweise nicht mehr als 6,5 A, noch weiter vorzugsweise nicht mehr als 6 A betragen und optimalerweise im Bereich zwischen 4 A und 6 A liegen sollten, um konzentrierten Dampf bei Vermeidung von Überhitzung zu gewährleisten.

Die Förderrate der Dochtstruktur 19 ist wiederum optimal an die an die Verdampfungsrate des Heizkörpers 60 angepasst, so dass jederzeit ausreichend Flüssigkeit nachgefördert werden kann und ein Leerlaufen des Bereichs vor dem Heizkörper 60 vermieden wird.

Die Verdampfervorrichtung 1 ist vorzugsweise auf der Grundlage von MEMS-Technologie, insbesondere aus Silizium, gefertigt und daher vorteilhaft ein Mikro-Elektro-Mechanisches System.

Vorgeschlagen wird nach dem zuvor Gesagten vorteilhaft ein Schichtaufbau bestehend aus einem vorteilhaft mindestens auf der Einlassseite 61 planaren Heizkörper 60 auf Si-Basis und einer oder mehrerer darunter liegender Kapillarstrukturen 19 mit vorteilhaft unterschiedlicher Porengröße. Die direkt an der Einlassseite 61 des Heizkörpers 60 angeordnete Dochtstruktur 19 verhindert die Bildung von Blasen an der Einlassseite 61 des Heizkörpers 60, da Gasblasen eine weitere Förderwirkung unterbinden und gleichzeitig zu einer (lokalen) Überhitzung des Heizkörpers 60 aufgrund fehlender Kühlung durch nachströmendes Liquid führen.

## Patentansprüche

1. Verfahren zur Herstellung einer Mehrzahl von elektrisch beheizbaren Heizkörpern (60) für eine Verdampfervorrichtung für einen Inhalator, insbesondere für ein elektronisches Zigarettenprodukt, und mit mindestens einer Durchgangsöffnung (62) zum Verdampfen von in der Durchgangsöffnung (62) befindlicher Flüssigkeit, **gekennzeichnet durch**
- Bereitstellen eines plattenförmigen Halbleitermaterials (103),
- Einarbeiten einer Mehrzahl von sich in Richtung der Flächennormale des Halbleitermaterials (103) durch das Halbleitermaterial (103) erstreckenden Durchgangsöffnungen (62),
- Trennen des Halbleitermaterials (103) entlang wenigstens einer Trennlinie (104) zur Bereitstellung der Mehrzahl von Heizkörpern (60), und
- Anordnen einer wenigstens der Anzahl von Heizkörpern (60) entsprechenden Anzahl von Dichtungselementen (7, 7a, 7b), so dass jede Durchgangsöffnung (62) von einem Dichtungselement (7, 7a, 7b) umfänglich umschlossen ist.

2. Verfahren nach Anspruch 1, **dadurch gekennzeichnet, dass**
- vor dem Trennen des Halbleitermaterials (103) die Dichtungselemente (7, 7a, 7b) angeordnet werden.

3. Verfahren nach Anspruch 1 oder 2, **gekennzeichnet durch**,
- das Anordnen der Dichtungselemente (7, 7a, 7b) in einem flüssigen Zustand.

4. Verfahren nach einem der vorangehenden Ansprüche 1 bis 3, **dadurch gekennzeichnet, dass**
- die Dichtungselemente (7, 7a, 7b) beim Anordnen stoffschlüssig mit dem Halbleitermaterial (103) und/oder dem Heizkörper (60) verbunden werden.

## Claims

1. Method for manufacturing a plurality of electrically heatable heating bodies (60) for an evaporator device for an inhaler, in particular for an electronic cigaratte product, and having at least one passage opening (62) for evaporating liquid contained in the passage opening (62), **characterised by**
- providing a plate-like semiconductor material (103),
- incorporating a plurality of passage openings (62) extending in the direction of the surface normal of the semiconductor material (103) through the semiconductor material (103),
- separating the semiconductor material (103) along at least one separation line (104) to provide the plurality of heating bodies (60), and
- arranging a number of sealing members (7, 7a, 7b) corresponding to at least the number of heating bodies (60) in such a manner that each passage opening (62) is circumferentially enclosed by a sealing member (7, 7a, 7b).

2. Method according to claim 1, **characterised in that**
- the sealing members (7, 7a, 7b) are arranged before the semiconductor material (103) is separated.

3. Method according to claim 1 or 2, **characterised by**
- arranging the sealing members (7, 7a, 7b) in a liquid state.

4. Method according to any of the preceding claims 1 to 3,
**characterised in that**
- the sealing members (7, 7a, 7b) are substance bonded to the semiconductor material (103) and/or the heating body (60) during the arranging.

## Revendications

1. Procédé de fabrication d'une pluralité d'éléments chauffants (60) chauffables électriquement pour un dispositif d'évaporation pour un inhalateur, en particulier pour un produit de cigarette électronique, et comportant au moins une ouverture de passage (62) pour vaporiser du liquide présent dans l'ouverture de passage (62), **caractérisé par**
- la fourniture d'un matériau semi-conducteur (103) en forme de plaque,
- le ménagement d'une pluralité d'ouvertures de passage (62) s'étendant à travers le matériau semi-conducteur (103), dans le sens de la normale de surface du matériau semi-conducteur (103),
- la séparation du matériau semi-conducteur (103) le long d'au moins une ligne de séparation (104) pour fournir la pluralité d'éléments chauffants (60),
et
- l'agencement d'un nombre d'éléments d'étanchéité (7, 7a, 7b) correspondant au moins au nombre d'éléments chauffants (60), de sorte que chaque ouverture de passage (62) soit entourée sur son pourtour par un élément d'étanchéité (7, 7a, 7b).

2. Procédé selon la revendication 1, **caractérisé en ce que**
- les éléments d'étanchéité (7, 7a, 7b) sont agencés avant la séparation du matériau semi-conducteur (103).

3. Procédé selon la revendication 1 ou 2, **caractérisé par**
- l'agencement des éléments d'étanchéité (7, 7a, 7b) dans un état liquide.

4. Procédé selon l'une des revendications précédentes 1 à 3, **caractérisé en ce que**
- les éléments d'étanchéité (7, 7a, 7b) sont reliés par liaison de matière avec le matériau semi-conducteur (103) et/ou l'élément chauffant (60) lors de l'agencement.
